(19) Europäisches Patentamt
European Patent Office
Office européen des brevets

(11) Publication number: **0 114 456**
**A2**

# (12) EUROPEAN PATENT APPLICATION

(21) Application number: 83304962.0

(22) Date of filing: 26.08.83

(51) Int. Cl.³: C 07 D 261/14
A 01 N 47/28

(30) Priority: 27.08.82 JP 149545/82

(43) Date of publication of application:
01.08.84 Bulletin 84/31

(84) Designated Contracting States:
BE CH DE FR IT LI NL SE

(71) Applicant: SHIONOGI & CO., LTD.
12, Dosho-machi 3-chome Higashi-ku
Osaka 541(JP)

(72) Inventor: Sumimoto, Shinzaburo
35-18, Iwazono-cho
Ashiya-shi Hyogo(JP)

(72) Inventor: Ishizuka, Ichiro
4-5-8, Tokiwadai
Toyono-gun Osaka(JP)

(72) Inventor: Ide, Kinya
170, Hirai-cho
Kusatsu-shi Shiga(JP)

(72) Inventor: Ueyama, Yoshito
963-20, Kamigasa-cho
Kusatsu-shi Shiga(JP)

(74) Representative: Bizley, Richard Edward et al,
BOULT, WADE & TENNANT 27 Furnival Street
London EC4A 1PQ(GB)

(54) **Acylated isoxazolyl ureas, their preparation, herbicidal formulations containing the same, and a herbicidal method.**

(57) New compounds are provided of formula:

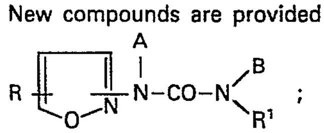 ;

in which A is hydrogen or $C_1-C_{10}$ acyl,
B is hydrogen, $C_1-C_5$ alkyl or $C_1-C_{10}$ acyl,
R is $C_3-C_4$ alkyl, and
$R^1$ is hydrogen, $C_1-C_5$ alkyl, $C_2-C_5$ alkenyl, $C_1-C_5$ alkoxy or phenyl, provided that either A or B is $C_1-C_{10}$ acyl. These compounds are useful as herbicides and may be prepared by several routes.

EP 0 114 456 A2

TITLE MODIFIED
see front page

## ACYLATED ISOXOZYLYUREAS, THEIR PREPARATION, HERBICIDAL FORMULATIONS CONTAINING THE SAME, AND A HERBICIDAL METHOD

The present invention relates to certain acylated isoxazolylureas.

U.S. Patent Specification No. 4062861 describes certain isoxazolylureas and 1,1-dimethyl-3-(5-t-butyl-isoxazolyl)urea (generic name: isouron) has been developed as an excellent herbicide. This invention is based upon the discovery of acylated osoxazolylureas which are also useful as herbicides.

According to the invention there is provided a compound of the formula:

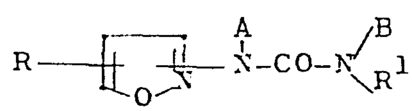

wherein R is $C_3$-$C_4$ alkyl,

$R^1$ is hydrogen, $C_1$-$C_5$ alkyl, $C_2$-$C_5$ alkenyl, $C_1$-$C_5$ alkoxy or phenyl,

A is hydrogen or $C_1$-$C_{10}$ acyl, and

B is hydrogen, $C_1$-$C_5$ alkyl or $C_1$-$C_{10}$ acyl;

provided that either A or B is $C_1$-$C_{10}$ acyl.

In the above formula:-

"Alkyl" includes methyl, ethyl, propyl, isopropyl, butyl, isobutyl, s-butyl, t-butyl and pentyl;
"alkenyl" includes vinyl, propenyl, butenyl and pentenyl;
"alkoxy" includes methoxy, ethoxy, propoxy, isopropoxy, butoxy, isobutoxy and pentyloxy;
"acyl" includes $C_1$-$C_5$ alkanoyl (e.g. formyl, acetyl, propionyl, butyryl, valeryl), cyclopropanecarbonyl and benzoyl in which the benzene ring may be optionally substituted by one or two substituents selected from $C_1$-$C_3$ alkyl, $C_1$-$C_3$ alkoxy, halogen, trifluoromethyl and nitro; and
"halogen" includes fluorine, chlorine, bromine and iodine.

The objective compounds (I) may be prepared by the following Routes, which represent processes included in the present invention:

Route A:

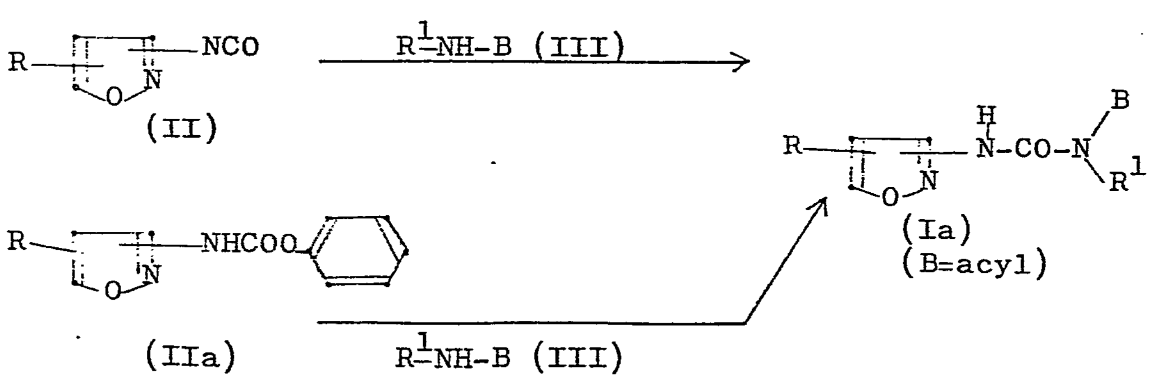

(II)
(IIa)
(Ia)
(B=acyl)

Route B:

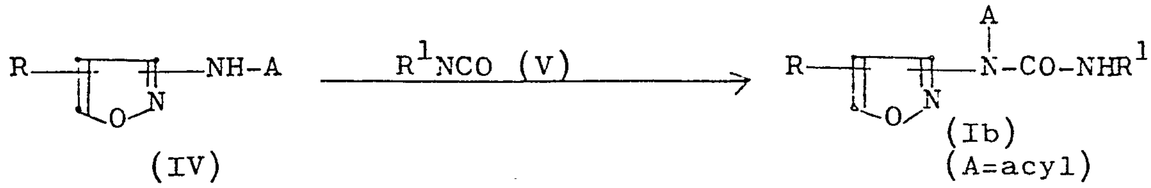


(IV)

(Ib)
(A=acyl)

Route C:

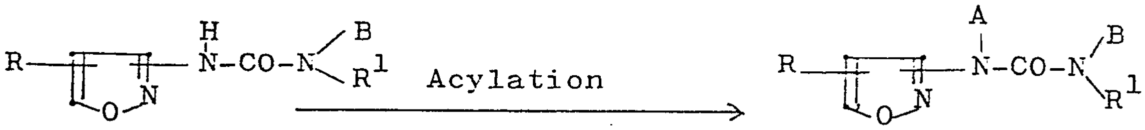


(VI)

(Ic)
(A=acyl; B=alkyl; $R^1 \neq$ hydrogen)

Route A:

Reaction of an isoxazolyl isocyanate (II) with an acylamide (III) may be performed in an appropriate solvent such as benzene, toluene, xylene, chloroform, ethyl acetate, dimethylsulfoxide or acetone, at about 30 to 150°C, preferably 60 to 120°C.

The starting isoxazoyl isocyanate (II) can be prepared, for example, by the following scheme:

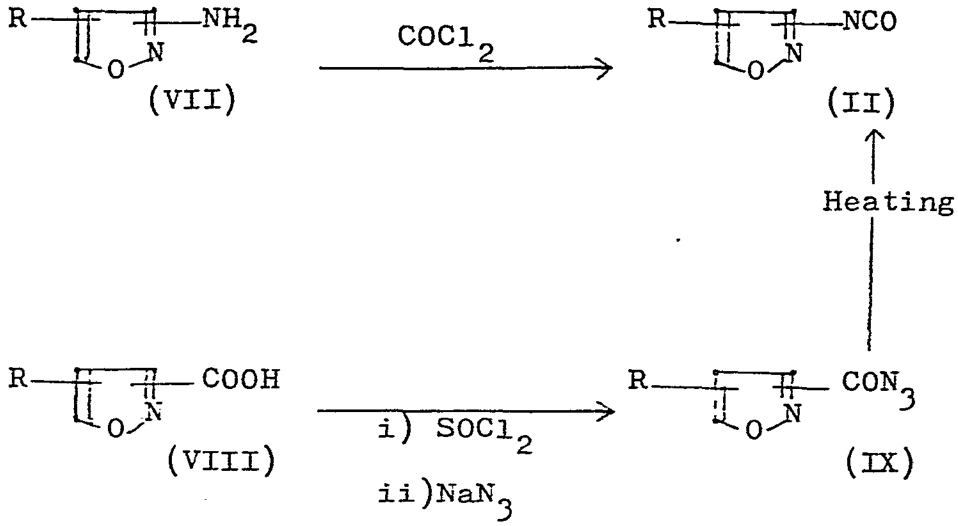


(VII) (II) (VIII) (IX)

The reaction mixture containing the isocyanate (II) prepared from the amine (VII) or azide (IX) may also be used as a starting material in the process of the invention.

As an alternative method in this Route, a phenyl carbamate (IIa) may be reacted with an acylamide (III). The reaction may be performed in a solvent such as benzene, toluene or xylene at about the boiling point of the solvent used. Furthermore, the starting ester (IIa) can be prepared by reacting an amine (VII) with phenyl chlorocarbonate.

Route B:

Reaction of acylamide (IV) with an isocyanate (V) in this Route may be performed in a similar manner to the reactions of Route A.

The starting acylamides (IV) may be prepared by

acylating the corresponding amines (VII):

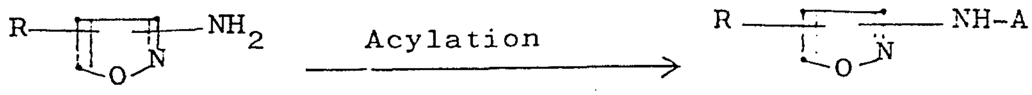

(VII)

(IV)

(in which A is $C_1$-$C_{10}$ acyl)

Route C:

Acylation of the starting isoxazolylurea (VI) may be performed in the presence of a base such as an alkali metal hydride, sodium amide, or an alkali metal alkoxide in an appropriate solvent such as dimethylformamide, chloroform, dimethylsulfoxide, benzene, toluene or hexamethylphosphortriamide at about 0°C to 200°C, preferably 30 to 102°C. Acylation using a substituted benzoyl chloride is preferred in this Route.

The invention will now be further described and illustrated by way of the following Examples.

The starting 3-isoxazolylcarbonyl azides and 3-isoxazolyl isocyanates may be prepared by processes already known from Japanese Unexamined Pat. Publns. Nos. 75063/1976 and 75064/1976.

The 5-isoxazolylcarbonyl azides and 5-isoxazolyl isocyanates are also available by using the same processes.

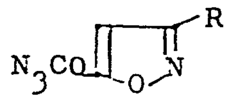 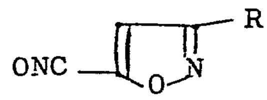

| R | mp (°C) | bp (°C/mmHg) |
|---|---|---|
| $CH_3$ | 107-108 (dec) | 70-71/20.5 |
| $(CH_3)_2CH$ | 24-25 | 84-85/14.5 |
| $(CH_3)_3C$ | 57-58 | 96-97/24 |

The starting 3-acylaminoisoxazoles may be prepared by the processes disclosed in Japanese Unexamined Pat. Pblns. Nos. 31039/1975 and 59272/1979. The 5-acylaminoisoxazoles are also available by the same processes.

The other starting 3-isoxazolylureas are preparable by the processes disclosed in Japanese Unexamined Pat. Publns. Nos. 31039/1975, 63170/1976, 46779/1979 and 59272/1979 and Japanese Pat. Publn. No. 44723/1979. The 5-isoxazolylureas are also preparable by the processes disclosed in Japanese Unexamined Pat. Publn. No. 41431/1976.

Example 1.

To 3-t-butyl-5-isoxazolyl isocyanate (2.39 g) are added toluene (29 ml) and N-methylformamide (1.70g), and the resultant mixture is refluxed for 1 hour. The reaction mixture is concentrated in vacuum, and the residue is chromatographed on a column of silica gel to give 1-methyl-1-formyl-3-(3-t-butyl-5-isoxazolyl)urea (3.02 g). The yield is 93.0 %. This substance is recrystallized from

10 % benzene-cyclohexane to give colorless prisms melting at 113 to 114°C.

Anal.Calcd. for $C_{10}H_{15}N_3O_3$: C, 53.32; H, 6.71; N, 18.65 (%) Found: C, 53.81; H, 6.63; N, 18.38 (%).

The compounds described in the following Examples are prepared as defined above.

Example 2.

1-Methyl-1-formyl-3-(3-isopropyl-5-isoxazolyl)urea. The yield is 90.0 %. Colorless needles melting at 57.0 to 58.0°C.

Anal.Calcd. for $C_9H_{13}N_3O_3$: C, 51.17; H, 6.20; N, 19.90 (%). Found: C, 51.18; H, 6.05; N, 19.97 (%).

Example 3.

1-Ethyl-1-formyl-3-(3-t-butyl-5-isoxazolyl)urea. The yield is 86.4 %. Slightly yellow oil. IR ($CCl_4$): 1728, 1372, 1232 $cm^{-1}$.

Example 4.

1-n-Propyl-1-formyl-3-(3-t-butyl-5-isoxazolyl)urea. The yield is 83.4 %. Slghtly yellow liquid. IR ($CCl_4$): 1728, 1696, 1608 $cm^{-1}$.

Example 5.

1-n-Butyl-1-formyl-3-(3-t-butyl-5-isoxazolyl)urea. The yield is 83.4 %. Slightly yellow liquid. IR ($CCl_4$): 1730, 1610, 1532 $cm^{-1}$.

Example 6.

(a) 5-t-Butyl-3-ethoxycarbonylisoxazole (9.86 g) is hydrolyzed with 5 % aqueous sodium hydride to give 5-t-butyl-isoxazole-3-carboxylic acid, which is treated with thionyl chloride under refluxing to give the corresponding acid chloride. The acid chloride is treated with aqueous sodium azide in benzene (100 ml) at about 5°C to give a solution of 5-t-butyl-3-isoxazolylcarbonyl azide in benzene, which is heated at 80°C for 5 hours and mixed with N-methylformamide (5.91 g). The resultant mixture is refluxed for 3 hours, allowed to stand at room temperature overnight and concentrated in vacuum. The residue is chromatographed on a column of silica gel to give 1-methyl-1-formyl-3-(5-t-butyl-3-isoxazoly)urea (8.38 g) as crystals. The yield is 74.4 %. This substance is repeatedly recrystallized from cyclohexane to give colorless plates melting at 99 to 100°C.

Anal. Calcd. for $C_{10}H_{15}N_3O_3$: C, 53.32; H, 6.71; N, 18.65 (%). Found: C, 53.56; H, 6.62; N, 18.61 (%).

(b) A mixture of 5-t-butyl-3-aminoisoxazole (56.1 g) and toluene (1200 ml) is stirred at 40°C to give a solution, which is cooled at 25 to 30°C. After hydrogen chloride gas (16.0 g) is introduced, phosgene (49.5 g) is incorporated into the solution in about half an hour. After finishing the incorporation, the resultant mixture is stirred at 55 to 60°C for 5.5 hours and at 70°C for half an hour. The toluene (about 800 ml) is evaporated under slightly reduced pressure,

and fresh toluene (600 ml) and N-methylformamide (47.3 g) are added to the residue. The mixture is refluxed for 5 hours, concentrated in vacuum, and the residue is dissolved in ethyl ether (1 L). The ethyl ether layer is washed twice with water (300 ml), and the washings are again extracted with ethyl ether. The ethyl ether layers are combined, dried over anhydrous sodium sulfate, and concentrated in vacuum to give 1-methyl-1-formyl-3-(5-t-butyl-3-isoxazolyl)urea (87.6 g). The yield is 97.2 %.

Example 7.

5-t-Butyl-3-ethoxycarbonylisoxazole (9.86 g) is treated as in Example 6 (1) to give a solution of 5-t-butyl-3-isoxazolylcarbonyl azide in benzene (100 ml), via the corresponding acid chloride. The solution is mixed with N-methylformamide (5.91 g), heated at 80°C for 8.5 hours, allowed to stand at room temperature overnight and concentrated in vacuum. The residue is purified by using chromatography on a column of silica gel to give 1-methyl-1-formyl-3-(5-t-butyl-3-isoxazolyl)urea (9.70 g) as crystals. The yield is 86.1 %.

Examples 8-15.

The following compounds are prepared in the same manner as described in Example 6.

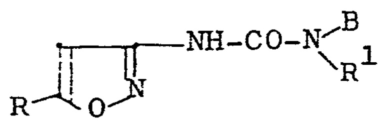

| Ex.No. | R | $R^1$ | B | mp(°C)/IR($cm^{-1}$) | yield(%) |
|---|---|---|---|---|---|
| 8 | i-Pr | Me | CHO | 103.5-105 | 75.6 |
| 9 | C-Pr | Me | CHO | 115 -116 | 73.1 |
| 10 | i-Bu | Me | CHO | 81 - 82 | 51.5 |
| 11 | t-Bu | Et | CHO | 98 - 99 | 65.6 |
| 12 | t-Bu | Pr | CHO | 1728, 1604, 1201 ($CCl_4$) | 62.9 |
| 13 | t-Bu | Bu | CHO | 1732, 1608, 1201 ($CCl_4$) | 68.0 |
| 14 | t-Bu | Me | $COCH_3$ | 68 - 69 | 63.3 |
| 15 | t-Bu | Me | $COCH_2CH_3$ | 91.0-91.5 | 59.6 |

Note: The abbreviations have the following significance: Me(Methyl), Et(Ethyl), Pr(Propyl), Bu(Butyl), i(iso), t(tertiary) and C(cyclo).

Example 16.

To N-(5-t-butyl-3-isoxazolyl)carbamic acid phenyl ester (1.30 g) are added benzene (10 ml) and N-methylformamide (0.89 g), and the resultant mixture is refluxed for 7 days. The reaction mixture is concentrated in vacuum, and the residue is purified by using the chromatography on a column of silica

gel to give 1-methyl-1-formyl-3-(5-t-butyl-3-isoxazolyl)urea (0.53 g) as crystals. The yield is 47 %.

Example 17.

To 5-isopropyl-3-acetylaminoisoxazole (4.96 g) is added methyl isocyanate (5.26 ml), and the resultant mixture is heated at 60°C in a sealed tube. Unreacted methyl isocyanate is distilled off from the reaction mixture, and the residue is purified by using chromatography on a column of silica gel to give 1-methyl-3-acetyl-3-(5-isopropyl-3-isoxazolyl)urea (5.55 g) as crystals. The yield is 83.5 %. The substance is recrystallized from 30 % benzene-cyclohexane to give colorless needles melting at 88.5 to 89.0°C.

Anal. Calcd. for $C_{10}H_{15}N_3O_3$: C, 53.32; H, 6.71; N, 18.65(%) Found: C, 53.32; H, 6.64; N, 18.66 (%).

Example 18-22.

The following compounds are prepared in the same manner as in Example 17.

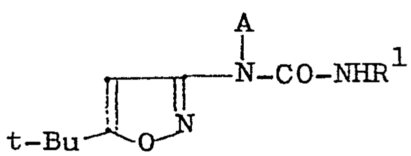

| Ex.No. | A | $R^1$ | mp(°C)/IR($cm^{-1}$) | Yield(%) |
|---|---|---|---|---|
| 18 | $CH_3$<br>$-COCHCH_2CH_2CH_3$ | Me | 1728, 1436,<br>1172 ($CCl_4$) | 52.6 |
| 19 | -CO 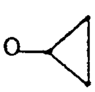 | Al | 90.0 - 90.5 | 9.7 |
| 20 | -CO 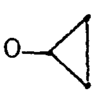 | Ph | 105 - 106 | 9.3 |

Note: The abbreviation has the following significance:

Al(Allyl), Ph(Phenyl).

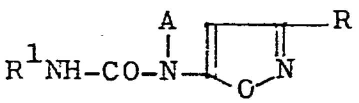

| Ex.No. | A | $R^1$ | R | mp(°C) | Yield(%) |
|---|---|---|---|---|---|
| 21 | $COCH_3$ | Me | i-Pr | 80 - 81 | 41.4 |
| 22 | $COCH_3$ | Me | t-Bu | 105 - 106 | 26.7 |

Example 23.

To 5-t-butyl-3-(cyclopropanecarbonyl)aminoisoxazole (3.15 g) are added methyl isocyanate (2.7 ml) and triethylamine (3 drops) and the resultant mixture is allowed to stand at room temperature. Unreacted methyl isocyanate is distilled off from the reaction mixture, and the residue is recrystallized from 50 % benzene-cyclohexane to give 1-methyl-3-cyclopropanecarbonyl-3-(5-t-butyl-3-isoxazolyl)urea (2.98 g) as colorless needles melting at 122.5 to 123°C. The yield is 74.4 %.

Anal. Calcd. for $C_{13}H_{19}N_3O_3$: C, 58.85; H, 7.22; N, 15.84 (%). Found: C, 58.66; H, 7.34; N, 15.77 (%).

Example 24-27.

The following compounds are prepared in the same manner as in Example 23.

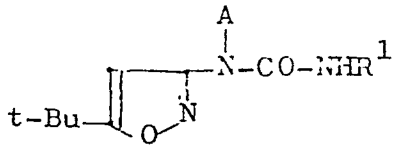

| Ex.No. | A | $R^1$ | mp(°C)/IR($cm^{-1}$) | Yield(%) |
|---|---|---|---|---|
| 24 | CHO | Me | 1728, 1592, 1440 ($CHCl_3$) | 29.4 |
| 25 | $COCH_3$ | Me | 119 - 121 | 70.0 |
| 26 | $COCH_2CH_3$ | Me | 108 - 109 | 72.8 |
| 27 | CO– 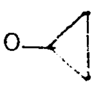 | Bu | 62 - 63 | 54.6 |

Example 28.

The following compound is prepared in the same manner as in Example 23.

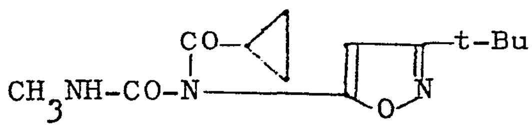

| mp(°C) | Yield(%) |
|---|---|
| 93 - 94 | 50.2 |

Example 29.

To a solution of 1,1-dimethyl-3-(5-t-butyl-3-isoxazolyl)-urea (2.96 g) in dry dimethylformamide (14 ml) is added 50 % sodium hydride (mineral oil suspension) (0.67 g) at room temperature with stirring, and the resultant mixture is stirred at 60°C for 15 minutes. 4-Methoxybenzoyl chloride (2.87 g) is added dropwise to the mixture with cooling below 5°C, the mixture being stirred at room temperature for 1 hour. Methanol (5 ml) is added to the mixture, which is allowed to stand at room temperature overnight. The reaction mixture is concentrated in vacuum, and the residue is mixed with water (25 ml) and

shaken with benzene. The benzene layer is washed with aqueous saturated sodium bicarbonate and water in order, dried over anhydrous sodium sulfate, and concentrated in vacuum. The residue is purified by using chromatography on a column of silica gel to give 1,1-dimethyl-3-(4-methoxybenzoyl)-3-(5-t-butyl-3-isoxazolyl)urea (4.13 g) as crystals. The yield is 85.3 %. This substance is recrystallized from benzene to give colorless needles melting at 138 to 139°C.

Anal. Calcd. for $C_{18}H_{23}N_3O_4$: C, 62.59; H, 6.71; N, 12.17 (%). Found: C, 62.54; H, 6.70; N, 12.19 (%).

Examples 30-42.

The following compounds are prepared in the same manner as in Example 29.

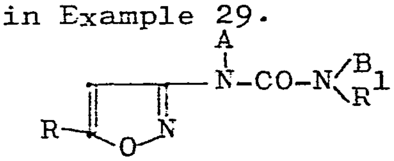

| Ex. No. | R | A | B | $R^1$ | mp(°C)/IR($cm^{-1}$) | Yield(%) |
|---|---|---|---|---|---|---|
| 30 | t-Bu | -CO-Ph | Me | Me | 109 - 110 | 68.2 |
| 31 | t-Bu | -CO-Ph | Me | Bu | 70.5 - 74.0 | 73.4 |
| 32 | t-Bu | -CO-Ph | Me | Al | 62.0 - 67.0 | 87.9 |
| 33 | i-Pr | -CO-Ph | Me | Me | 73.0 - 74.0 | 69.5 |
| 34 | i-Bu | -CO-Ph | Me | Me | 118.5 - 119.5 | 59.5 |
| 35 | t-Bu | 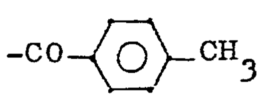 | Me | Me | 135.5 - 136.0 | 90.3 |

| Ex. No. | R | A | B | $R^1$ | mp(°C)/IR($cm^{-1}$) | Yield(%) |
|---|---|---|---|---|---|---|
| 36 | t-Bu | 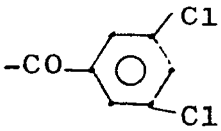 | Me | Me | 98.5 - 99.5 | 75.0 |
| 37 | t-Bu | 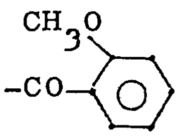 | Me | Me | 110 - 111.5 | 90.7 |
| 38 | t-Bu | 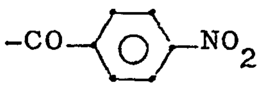 | Me | Me | 164.0 - 164.5 | 75.4 |
| 39 | t-Bu | 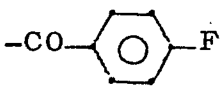 | Me | Me | 123.5 - 124.5 | 79.8 |
| 40 | t-Bu | 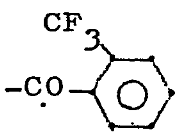 | Me | Me | 143.0 - 143.5 | 79.9 |
| 41 | t-Bu | -CO-Ph | Me | Me-O | 1710, 1680, 1438 ($CHCl_3$) | 32.1 |
| 42 | t-Bu | 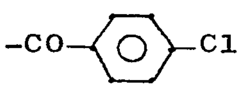 | Me | Me | 160.5 - 161.5 | 65.3 |

Example 43-44.

The following compounds are prepared in the same manner as in Example 29.

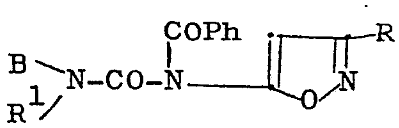

| Ex.No. | B | $R^1$ | R | mp(°C)/IR($cm^{-1}$) | Yield(%) |
|---|---|---|---|---|---|
| 43 | Me | Me | t-Bu | 101.5 - 102.5 | 74.8 |
| 44 | Me | Me | i-Pr | 1708, 1465, 1395 ($CHCl_3$) | 80.6 |

The acylated isoxazolylureas (I) of the present invention show excellent herbicidal activity at small application rates against various weeds and grasses such as, for example, the following:

Digitaria adscendens (large crabgrass),
Setaria viridis (green foxtail),
Echinochloa crusgalli (barnyard grass),
Amaranthus viridis (green amaranth),
Chenopodium album (smooth pigweed),
Cyperus micro-iria (flat-sedge),
Mollugo stricta (carpet-weed),
Stellaria media (common chickweed),
Sagina japonica (pearlwort),
Stellaria Alsine (bog-stichwort),
Capsella Bursa-pastoris (Shepherd's purse),
Alopecurus aequalis (water foxtail),
Polygonum longisetum (smartweed),
Trigonotis peduncularis (-),
Gnaphalium affine (cottonweed),
Echinochloa oryzicola (barnyard grass),
Monchoria vaginalis (monochoria),
Cyperus difformis (umbrella plant),
Rotala indica (tooth cup), and
Dopatrium junceum (-).

In addition, when used at an application rate of from 1.25 to 10 g/are the present compounds either produce almost no chemical damage to various crops, such as, e.g. corn,

sugar cane, sorghum, soybean or cotton, or if there is any such effect, only very slight harm to an extent that permits easy recovery. Furthermore, the compounds (I) of the present invention are generally applicable as selective or non-selective herbicides over various types of crop land including ploughed fields, orchards, tea plantations, mulberrry farms, fallow lands and pastures and non-crop land including railbeds, roads, lawns, factory sites, dry riverbeds, residential quarters, park green districts, forest lands, prepared lands and vacant lands.

The present compounds (I) are harmless and safe to humans, domestic animals and poultry and exhibit very low toxicity towards fishes and shells. Thus, the herbicides of the present invention may be used with high safety and also are free from the production of residual poisoning in the soil.

The compounds (I) may be used together with already known herbicides, such as, e.g., diuron, atrazine, prometryn, alachlor or trifluralin, for the purpose of enlarging the herbicidal spectrum in the resulting formulation and realizing summing or synergistic herbicidal effects.

The herbicides of the present invention are applicable to many and various kinds of soil such as

sandy loam soils, loamy sands, clay soils and special soils apt to have an influence over the appearance of herbicidal activity, such as montmorillonite soil.

The invention includes a herbicidal formulation comprising a compound of the invention, optionally together with an agricultural carrier, diluent and/or excipient.

The herbicidal formulations of the present invention may be prepared by, for example, mixing the effective isoxazolylurea (I) with an appropriate solid and/or liquid carrier, possibly in combination with further supplements such as emulsifiers, stabilizers, dispersants, suspending agents, spreaders, penetrants, or wetting agents. The resultant mixture may be converted into any desired type of formulation such as an emulsion, wettable powder, granules, dust, pills or a solution. Examples of the carriers which may be used include solid carriers (e.g. clay, talc, diatomaceous earth or bentonite) and liquid carriers (e.g. water, alcohols, acetone, benzene, toluene, xylene, solvent naphtha or cyclohexane). The herbicides of this invention ma be used in combination with other agricultural chemicals (e.g. insecticides, fungicides or other herbicides), manuring ingredients (e.g. ammonium sulfate or urea) or soil-treating agents.

The herbicidal activity of the present herbicides

may be illustrated by the following Experiments. The quoted test compound numbers therein correspond to the numbers of the aforementioned Examples.

The following compounds were used as controls:

Control 1:

Diuron[1,1-domethyl-3-(3,4-dicholophenyl)urea], disclosed in U.S. Patent No. 2,768,971.

Control 2:

1-Methyl-1-formul-3-(3,4-dichlorophenyl)urea, disclosed in U.S. Patent No. 2,762,695.

Experiment 1.

a) Test Method:

(1) Post emergence test:

In polyethylene cups (diameter : 9 cm) charged with sandy loam soil, seeds of the test plants were sown by species. After sowing, the seeds were covered with sandy loam soil to about 5 mm thickness. Test plants were cultivated for 10 days after seeding.

An aqueous suspension of the test compound made by using as a spreader Tween 20 (Atlas Powder Co.) at a concentration of 100 ppm was applied over the young plants. Application rate of the test compound was 10 g/are and 30 g/are, and the aqueous suspension (water dilution : 10 L/are) was applied by a hand-sprayer. Administration was performed at 25°C in a greenhouse in

natural sunlight. Survival ratio was appraised 3 weeks after application.

(2) Pre-emergence test:

An aqueous suspension of the test compound was applied over the surface of the sandy loam soil soon after sowing the seeds of the test plants. Administration and appraisal were performed as described above.

b) Method of the appraisal:

Results were assessed by observing the plants with the naked eye for some time after test compound application and calculating the survival ratio of the number of living plants 3 weeks after application. The appraisal was marked in six degrees of survival ratio as follows:

| Survival ratio of the plant tested | Mark(s) |
|---|---|
| Not more than 10 % | 5 |
| 11 - 25 % | 4 |
| 26 - 50 % | 3 |
| 51 - 75 % | 2 |
| 76 - 90 % | 1 |
| Not less than 91 % | 0 |

c) Result:

Table 1

| Compd. No. | Appln. rate (g/a) | Post-emergence test | | | | | | Pre-emergence test | | | | | |
|---|---|---|---|---|---|---|---|---|---|---|---|---|---|
| | | A | B | C | D | E | F | A | B | C | D | E | F |
| 1 | 10 | 0 | 0 | 0 | 5 | 3 | 5 | 0 | 0 | 1 | 4 | 4 | 5 |
| | 30 | 3 | 2 | 3 | 5 | 5 | 5 | 1 | 4 | 3 | 5 | 5 | 5 |
| 6 | 10 | 0 | 3 | 5 | 5 | 5 | 5 | 0 | 4 | 5 | 5 | 5 | 5 |
| | 30 | 3 | 5 | 5 | 5 | 5 | 5 | 0 | 5 | 5 | 5 | 5 | 5 |
| 8 | 10 | 0 | 0 | 1 | 5 | 1 | 5 | 0 | 0 | 2 | 3 | 1 | 5 |
| | 30 | 0 | 1 | 5 | 5 | 5 | 5 | 0 | 2 | 5 | 5 | 3 | 5 |
| 11 | 10 | 0 | 0 | 1 | 2 | 1 | 1 | 0 | 0 | 1 | 1 | 1 | 2 |
| | 30 | 0 | 1 | 4 | 5 | 5 | 5 | 0 | 0 | 4 | 3 | 1 | 3 |
| 13 | 10 | 0 | 0 | 1 | 4 | 1 | 4 | 0 | 0 | 0 | 1 | 0 | 0 |
| | 30 | 0 | 1 | 3 | 5 | 5 | 5 | 0 | 0 | 1 | 3 | 1 | 2 |
| 14 | 10 | 0 | 0 | 3 | 4 | 1 | 5 | 0 | 0 | 1 | 0 | 2 | 5 |
| | 30 | 0 | 0 | 3 | 5 | 1 | 5 | 0 | 1 | 3 | 4 | 5 | 5 |
| 17 | 10 | 0 | 1 | 1 | 5 | 1 | 5 | 0 | 1 | 2 | 4 | 1 | 5 |
| | 30 | 0 | 4 | 5 | 5 | 5 | 5 | 1 | 5 | 5 | 5 | 5 | 5 |
| 18 | 10 | 0 | 1 | 1 | 5 | 4 | 5 | 0 | 3 | 3 | 5 | 5 | 5 |
| | 30 | 1 | 1 | 1 | 5 | 5 | 5 | 0 | 5 | 5 | 5 | 5 | 5 |
| 19 | 10 | 0 | 0 | 0 | 5 | 0 | 1 | 0 | 0 | 1 | 5 | 2 | 4 |
| | 30 | 0 | 1 | 1 | 5 | 1 | 0 | 0 | 3 | 5 | 5 | 5 | 5 |
| 20 | 10 | 0 | 0 | 0 | 5 | 1 | 5 | 0 | 1 | 1 | 5 | 3 | 5 |
| | 30 | 0 | 1 | 1 | 5 | 4 | 5 | 0 | 2 | 5 | 5 | 5 | 5 |
| 23 | 10 | 1 | 5 | 5 | 5 | 5 | 5 | 1 | 5 | 5 | 5 | 5 | 5 |
| | 30 | 3 | 5 | 5 | 5 | 5 | 5 | 3 | 5 | 5 | 5 | 5 | 5 |
| 24 | 10 | 1 | 5 | 5 | 5 | 5 | 5 | 1 | 5 | 5 | 5 | 5 | 5 |
| | 30 | 3 | 5 | 5 | 5 | 5 | 5 | 2 | 5 | 5 | 5 | 5 | 5 |
| 25 | 10 | 1 | 5 | 5 | 5 | 5 | 5 | 1 | 5 | 5 | 5 | 5 | 5 |
| | 30 | 4 | 5 | 5 | 5 | 5 | 5 | 4 | 5 | 5 | 5 | 5 | 5 |

| Compd. No. | Appln.rate (g/a) | Post-emergence test | | | | | | Pre-emergence test | | | | | |
|---|---|---|---|---|---|---|---|---|---|---|---|---|---|
| | | A | B | C | D | E | F | A | B | C | D | E | F |
| 26 | 10 | 2 | 5 | 5 | 5 | 5 | 5 | 3 | 5 | 5 | 5 | 5 | 5 |
| | 30 | 5 | 5 | 5 | 5 | 5 | 5 | 4 | 5 | 5 | 5 | 5 | 5 |
| 27 | 10 | 0 | 0 | 0 | 5 | 1 | 5 | 0 | 0 | 0 | 2 | 1 | 5 |
| | 30 | 0 | 1 | 1 | 5 | 5 | 5 | 0 | 1 | 3 | 5 | 5 | 5 |
| 28 | 10 | 0 | 1 | 1 | 5 | 5 | 5 | 0 | 3 | 2 | 1 | 3 | 5 |
| | 30 | 1 | 3 | 3 | 5 | 5 | 5 | 1 | 3 | 5 | 2 | 5 | 5 |
| 29 | 10 | 0 | 1 | 3 | 5 | 5 | 5 | 0 | 4 | 5 | 4 | 5 | 5 |
| | 30 | 1 | 5 | 5 | 4 | 5 | 5 | 0 | 5 | 5 | 5 | 5 | 5 |
| 30 | 10 | 3 | 5 | 5 | 5 | 5 | 5 | 1 | 3 | 4 | 5 | 5 | 5 |
| | 30 | 5 | 5 | 5 | 5 | 5 | 5 | 1 | 4 | 5 | 5 | 5 | 5 |
| 31 | 10 | 0 | 3 | 4 | 5 | 5 | 5 | 0 | 0 | 1 | 3 | 5 | 5 |
| | 30 | 0 | 5 | 5 | 5 | 5 | 5 | 0 | 5 | 5 | 5 | 5 | 5 |
| 32 | 10 | 0 | 1 | 3 | 5 | 5 | 5 | 0 | 4 | 5 | 4 | 5 | 5 |
| | 30 | 2 | 5 | 5 | 5 | 5 | 5 | 2 | 5 | 5 | 5 | 5 | 5 |
| 33 | 10 | 0 | 3 | 3 | 5 | 5 | 5 | 0 | 4 | 5 | 5 | 5 | 5 |
| | 30 | 2 | 5 | 5 | 5 | 5 | 5 | 2 | 5 | 5 | 5 | 5 | 5 |
| 34 | 10 | 1 | 1 | 3 | 5 | 5 | 5 | 0 | 1 | 3 | 1 | 1 | 5 |
| | 30 | 2 | 4 | 5 | 5 | 5 | 5 | 1 | 5 | 5 | 5 | 5 | 5 |
| 36 | 10 | 1 | 5 | 3 | 5 | 5 | 5 | 0 | 4 | 5 | 4 | 4 | 5 |
| | 30 | 3 | 5 | 5 | 5 | 5 | 5 | 2 | 5 | 5 | 5 | 5 | 5 |
| 37 | 10 | 3 | 5 | 5 | 5 | 5 | 5 | 1 | 3 | 5 | 5 | 5 | 5 |
| | 30 | 4 | 5 | 5 | 5 | 5 | 5 | 2 | 5 | 5 | 5 | 5 | 5 |
| 38 | 10 | 0 | 1 | 0 | 1 | 0 | 5 | 0 | 2 | 0 | 2 | 2 | 5 |
| | 30 | 0 | 2 | 0 | 2 | 1 | 5 | 0 | 3 | 0 | 4 | 1 | 5 |
| 39 | 10 | 0 | 3 | 5 | 5 | 5 | 5 | 0 | 3 | 5 | 5 | 5 | 4 |
| | 30 | 3 | 2 | 5 | 5 | 5 | 5 | 1 | 5 | 5 | 5 | 5 | 5 |
| 40 | 10 | 3 | 4 | 5 | 5 | 5 | 5 | 1 | 3 | 3 | 5 | 5 | 5 |
| | 30 | 3 | 5 | 5 | 5 | 5 | 5 | 2 | 5 | 5 | 5 | 5 | 5 |

| Compd. No. | Appln.rate (g/a) | Post-emergence test | | | | | | Pre-emergence test | | | | | |
|---|---|---|---|---|---|---|---|---|---|---|---|---|---|
| | | A | B | C | D | E | F | A | B | C | D | E | F |
| 42 | 10 | 0 | 1 | 1 | 1 | 2 | 5 | 0 | 0 | 1 | 0 | 0 | 2 |
| | 30 | 0 | 1 | 2 | 5 | 5 | 5 | 0 | 2 | 2 | 3 | 2 | 4 |
| 43 | 10 | 2 | 5 | 5 | 5 | 5 | 5 | 1 | 4 | 5 | 5 | 5 | 5 |
| | 30 | 2 | 5 | 5 | 5 | 5 | 5 | 2 | 5 | 5 | 5 | 5 | 5 |
| 44 | 10 | 0 | 2 | 2 | 2 | 5 | 5 | 0 | 0 | 3 | 2 | 3 | 5 |
| | 30 | 1 | 5 | 5 | 5 | 2 | 5 | 1 | 1 | 3 | 3 | 5 | 5 |
| Control 1 | 10 | 0 | 2 | 4 | 5 | 1 | 5 | 0 | 1 | 2 | 1 | 1 | 4 |
| | 30 | 0 | 4 | 5 | 5 | 3 | 5 | 0 | 2 | 2 | 1 | 2 | 4 |

Note: A(wheat), B(barnyard grass), C(large crabgrass), D(oilseed rape), E(smartweed), F(green amaranth).

Thus, the tested compounds of the present invention (Compounds Nos. 1, 6, 8, 11, 13-14, 17-20, 23-34, 36-40, and 42-44) showed far more herbicidal activity both in the post-emergence test and in the pre-emergence test than a commercially available herbicide, diuron (Control 1).

Experiment 2.

a) Test method (Pre-emergence test):

In polyethylene cups (diameter : 13 cm) charged with sandy loam soil, the seeds of the test plants were sown by species. After sowing, the seeds were covered with sandy loam soil to about 5 mm thickness, and soon an aqueous suspension of the test compound made by using as a spreader Tween 20 at a concentration of 100 ppm was applied

over the surface of the sandy loam soil. Application rate of the test compound was 40, 20, 10, 5 and 2.5 g/are, and the aqueous suspension (water dilution : 10 L/are) was applied by a hand-sprayer. Administration was performed in natural sunlight.

b) Method of the appraisal:

Results were assessed by observing the plants with the naked eye for some time after test compound application, examining any chemical harm appearing on the plant body in accordance with the following six degrees and determining the corresponding mark(s).

| Degrees of the harm | mark(s) |
|---|---|
| Complete withering | 5 |
| Big damage | 4 |
| Moderate damage | 3 |
| Small damage | 2 |
| Slight damage | 1 |
| No damage | 0 |

c) Result:

Resuts are shown in Table 2.

Table 2

| Compd. No. | Appln. rate (g/n) | corn | wheat | upland rice | large crabgrass | soybean | cotton | sugar beet | smart-weed |
|---|---|---|---|---|---|---|---|---|---|
| 1 | 5 | 0 | 0 | 1 | 0 | 0 | 0 | 0 | 0 |
| | 10 | 0 | 2 | 3 | 3 | 2 | 1 | 1 | 3 |
| | 20 | 1 | 4 | 5 | 4 | 4 | 2 | 3 | 5 |
| | 40 | 3 | 5 | 5 | 5 | 5 | 3 | 4 | 5 |
| 6 | 2.5 | 0 | 0 | 1 | 1 | 0 | 0 | 2 | 1 |
| | 5 | 0 | 1 | 3 | 2 | 0 | 0 | 5 | 4 |
| | 10 | 1 | 3 | 4 | 4 | 3 | 0 | 5 | 5 |
| | 20 | 3 | 4 | 5 | 5 | 4 | 1 | 5 | 5 |
| 31 | 5 | 0 | 0 | 0 | 0 | 0 | 0 | 1 | 0 |
| | 10 | 0 | 2 | 4 | 4 | 3 | 2 | 4 | 5 |
| | 20 | 2 | 5 | 5 | 5 | 4 | 4 | 5 | 5 |
| | 40 | 4 | 5 | 5 | 5 | 5 | 5 | 5 | 5 |
| 39 | 2.5 | 0 | 0 | 2 | 2 | 0 | 0 | 2 | 2 |
| | 5 | 0 | 4 | 4 | 4 | 0 | 1 | 4 | 5 |
| | 10 | 3 | 4 | 5 | 4 | 2 | 2 | 5 | 5 |
| | 20 | 4 | 5 | 5 | 5 | 4 | 4 | 5 | 5 |
| 40 | 2.5 | 0 | 1 | 2 | 4 | 0 | 1 | 5 | 5 |
| | 5 | 1 | 4 | 4 | 5 | 1 | 2 | 5 | 5 |
| | 10 | 4 | 5 | 5 | 5 | 5 | 3 | 5 | 5 |
| | 20 | 5 | 5 | 5 | 5 | 5 | 5 | 5 | 5 |
| Control 2 | 5 | 0 | 0 | 0 | 0 | 0 | 0 | 0 | 0 |
| | 10 | 0 | 0 | 0 | 0 | 0 | 0 | 2 | 1 |
| | 20 | 0 | 0 | 1 | 2 | 0 | 0 | 5 | 1 |
| | 40 | 0 | 2 | 2 | 4 | 0 | 0 | 5 | 2 |

The compounds of the present invention are applicable as selective herbicides for crop plants. Thus, for example, Compound No. 6 was harmless to cotton but showed a powerful herbicidal activity against weeds such as large crabgrass and smartweed at an application rate of 10 g/are. On the other hand, Control 2 showed almost no herbicidal activity towards cotton and weeds at the same application rate.

Experiment 3.

a) Test method (Pre-emergence test):

In polyethylene cups (diameter 13 cm) charged with sandy loam soil, seeds of the test plants were sown by species. After sowing, the seeds were covered with sandy loam soil to about 5 mm thickness, and soon an aqueous suspension of the test compound made by using as a spreader Tween 20 at a concentration of 100 ppm was applied over the surface of the sandy loam soil. Application rate of the test compound was 5, 10, 20 and 40 g/are, and the aqueous suspension (water dilution : 10 L/are) was applied by a hand-sprayer. Administration was performed at 25°C in a greenhouse in natural sunlight.

b) Method of the appraisal:

Results were assessed by observing the plants with the naked eye for some time after test compound application, examining any chemical harm appearing on the plant

body in accordance with the following six degrees and determining the corresponding mark(s).

| Degrees of the harm | mark(s) |
|---|---|
| Complete withering | 5 |
| Big damage | 4 |
| Moderate damage | 3 |
| Small damage | 2 |
| Slight damage | 1 |
| No damage | 0 |

c) Result :

Results are shown in Table 3.

Table 3

| Compd. No. | Appln. rate (g/n) | corn | soybean | cotton | oilseed rape | barnyard grass | large crabgrass | green amaranth | smartweed | flat-sedge |
|---|---|---|---|---|---|---|---|---|---|---|
| 25 | 5 | 0 | 0 | 0 | 4 | 5 | 4 | 5 | 5 | 5 |
| | 10 | 0 | 1 | 2 | 5 | 5 | 5 | 5 | 5 | 5 |
| | 20 | 1 | 4 | 4 | 5 | 5 | 5 | 5 | 5 | 5 |
| | 40 | 4 | 5 | 5 | 5 | 5 | 5 | 5 | 5 | 5 |
| 26 | 5 | 0 | 0 | 2 | 5 | 5 | 4 | 5 | 5 | 5 |
| | 10 | 0 | 1 | 3 | 5 | 5 | 5 | 5 | 5 | 5 |
| | 20 | 2 | 4 | 5 | 5 | 5 | 5 | 5 | 5 | 5 |
| | 40 | 3 | 5 | 5 | 5 | 5 | 5 | 5 | 5 | 5 |

Thus, the tested compounds of the present invention (Compounds Nos. 25-26) were harmless to corn but showed a powerful herbicidal activity against various weeds such as barnyard grass, large crabgrass, green amaranth, smartweed and flat-sedge at an application rate of 10 g/are.

<u>Experiment 4</u>.

a) Test method (Pre-emergence test):

In a Wagner pot (1/5000 are) charged with loam soil, seeds of the test plants were sown by species. After sowing, the seeds were covered with the loam soil to about 5 mm thickness, and soon an aqueous suspension of the test compound made by using as a spread Tween 20 at a concentration of 100 ppm was applied over the surface of the loam soil. Application rate of the test compound was 1.25, 2.5, 5, 7.5, 10 and 15 g/are, and the aqueous suspension (water dilution : 10 L/are) was applied by a hand-sprayer. Administration was performed at 25°C in a greenhouse in natural sunlight.

b) Method of the appraisal:

The appraisal was made as in Experiment 2.

c) Result:

The results are shown in Table 4.

Table 4

| Compd. No. | Appln. rate (g/a) | corn | large crabgrass | barnyard grass | flat-sedge | green amaracanth | smartweed | smooth pigweed | common purslane | common chickweed |
|---|---|---|---|---|---|---|---|---|---|---|
| 6 | 1.25 | 0 | 3 | 2 | 3 | 4 | 5 | 4 | 5 | 5 |
| | 2.5 | 0 | 5 | 5 | 5 | 5 | 5 | 5 | 5 | 5 |
| | 5 | 0 | 5 | 5 | 5 | 5 | 5 | 5 | 5 | 5 |
| | 10 | 0 | 5 | 5 | 5 | 5 | 5 | 5 | 5 | 5 |
| 28 | 1.25 | 0 | 4 | 2 | 4 | 5 | 1 | 3 | 5 | 5 |
| | 2.5 | 0 | 5 | 2 | 5 | 5 | 5 | 5 | 5 | 5 |
| | 5 | 0 | 5 | 5 | 5 | 5 | 5 | 5 | 5 | 5 |
| | 10 | 1 | 5 | 5 | 5 | 5 | 5 | 5 | 5 | 5 |
| 40 | 5 | 0 | 3 | 3 | 3 | 4 | 2 | 5 | 5 | 5 |
| | 7.5 | 0 | 4 | 4 | 5 | 5 | 3 | 5 | 5 | 5 |
| | 10 | 0 | 5 | 4 | 5 | 5 | 4 | 5 | 5 | 5 |
| | 15 | 0 | 5 | 5 | 5 | 5 | 5 | 5 | 5 | 5 |

Thus, the tested compounds of the present invention (Compounds Nos. 6, 28 and 40) were harmless to corn but showed excellent herbicidal activities against various weeds such as large crabgrass, barnyard grass, flat-sedge, green amaracanth, smartweed, smooth pigweed, common purslane and common chickweed at several application rates.

The present invention includes a method of preventing or inhibiting the growth of weeds or grasses in an environment or of killing such weeds or grasses,

which method comprises administering to the environment a compound of the invention or a formulation of the invention.

Embodiments of the herbicidal formulations of the present invention are given in the following Examples.

Example A.

1-Methyl-1-formyl-3-(3-t-butyl-5-isoxazolyl)urea (10 parts by weight) is admixed evenly with fine powdery talc (90 parts by weight), whereby a dust is obtained.

Example B.

1-Methyl-3-(2-methylpentanoyl)-3-(5-t-butyl-3-isoxazolyl)urea (20 parts by weight), clay (50 parts by weight), talc (25 parts by weight) and a wetting agent, Emal (Registered trademark, Kao Soap Co.) (5 parts by weight) are admixed and pulverized, whereby a wettable powder is obtained.

Example C.

1-Methyl-1-butyl-3-benzoyl-3-(5-t-butyl-3-isoxazolyl)-urea (20 parts by weight) is dissolved in dimethylformamide (65 parts by weight) to give a solution, which is mixed with Sorpol (Registered trademark, Toho Chemical Ind.) (15 parts by weight) to give an emulsion.

CLAIMS:

1. A compound of the formula:

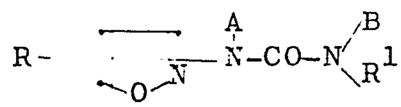

wherein R is $C_3$-$C_4$ alkyl,

$R^1$ is hydrogen, $C_1$-$C_5$ alkyl, $C_2$-$C_5$ alkenyl, $C_1$-$C_5$ alkoxy or phenyl,

A is hydrogen or $C_1$-$C_{10}$ acyl, and

B is hydrogen, $C_1$-$C_5$ alkyl or $C_1$-$C_{10}$ acyl;

provided that one of A and B is $C_1$-$C_{10}$ acyl.

2. A compound as claimed in claim 1, wherein R is 5-t-butyl.

3. A compound as claimed in claim 1 or claim 2, wherein A is hydrogen and B is $C_1$-$C_5$ alkanoyl.

4. A compound as claimed in claim 1 or claim 2, wherein A is cyclopropanecarbonyl and B is hydrogen.

5. A compound as claimed in claim 1 or claim 2, wherein A is halo-benzoyl and B is $C_1$-$C_5$ alkyl.

6. 1-Formyl-1-methyl-3-(5-t-butyl-3-isoxazolyl)-urea.

7. 1-Methyl-3-cyclopropanecarbonyl-3-(5-t-butyl-3-isoxazolyl)urea.

8. Dimethyl-3-(4-fluorobenzoyl)-3-(5-t-butyl-3-isoxazolyl)urea.

9. A process for preparing a compound as claimed

in claim 1, which process comprises either:

(a) reacting a compound of formula:

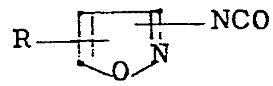

with an acylamide of formula $R^1$-NH-B; or

(b) reacting a compound of formula:

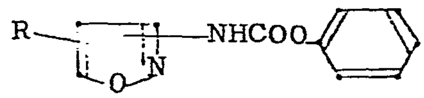

with an acylamide of formula $R^1$-NH-B; or

(c) reacting a compound of formula:

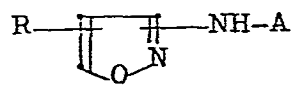

with an isocyanate of formula $R^1$NCO; or

(d) acylating a compound of formula:

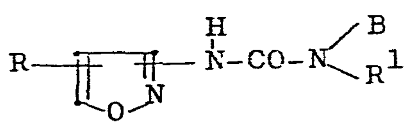

10. A herbicidal formulation comprising a compound as claimed in any one of claims 1 to 8, optionally together with an agricultural carrier, diluent and/or excipient.

11. A formulation as claimed in claim 10 also comprising a different herbicide, an agricultural chemical, a manuring ingredient, and/or a soil-treating agent.

12. A method of preventing or inhibiting the

growth of weeds or grasses in an environment or of killing such weeds or grasses, which method comprises administering to the environment a compound as claimed in any one of claims 1 to 8 or a formulation as claimed in claim 10 or claim 11.